# EUROPEAN PATENT APPLICATION

(11) **EP 2 747 199 A1**
(43) Date of publication of application: **25.06.2014**
(21) Application number: 13186791.3
(22) Date of filing: 01.10.2013
(51) Int. Cl.: H01Q 1/27, C08J 9/22, H01Q 1/38

(54) **Antenna and method for manufacturing antenna**

(30) Priority: 21.12.2012 KR 20120150397
(71) Applicant: Samsung Electronics Co., Ltd, Gyeonggi-do 443-742 (KR)
(72) Inventor: Ko, Byung Hoon, 446-712 Gyeonggi-do (KR); Park, Sang Yun, 446-712 Gyeonggi-do (KR); Kim, Youn Ho, 446-712 Gyeonggi-do (KR); Hong, Young Jun, 446-712 Gyeonggi-do (KR); Park, Kun Kook, 446-712 Gyeonggi-do (KR); Shin, Kun Soo, 446-712 Gyeonggi-do (KR)
(74) Representative: Grünecker, Kinkeldey, Stockmair & Schwanhäusser

(57) **Abstract**

An antenna and a method of manufacturing the antenna are provided. The antenna may include an antenna surface, a ground plane, and an air layer comprising a porous structure.

## Description

### BACKGROUND

### 1. Field

The following description relates to an antenna and a method for manufacturing the antenna. For example, the antenna is an antenna supported using an air layer that is stably structured.

### 2. Description of Related Art

A physiological signal measured from a body of a user may continuously be monitored throughout a patient's daily routine for diagnosis of diseases or management of a user's activity. A sensor is typically attached to the skin of the user to monitor the physiological signal of the user. Such a sensor may monitor electrocardiogram (ECG), electromyogram (EMG), electroencephalogram (EEG), galvanic skin response (GSR), electrooculogram (EOG), a body temperature, pulses, a blood pressure, a human body motion, or other physiological signals.

Recent advances in technology have provided a need for connecting an antenna to the sensor that is capable of wireless data transmission in order to transmit signals in real time. Typically, such an antenna applies a wireless body area network (WBAN) technology that forms an inter-node network by being implanted in or attached on the user's body. The antenna may be divided into an antenna layer, an air layer, and a ground layer. The antenna typically collects the biomedical data by generating radiated power that is concentrated on the user's body.

However, due to the air layer being filled with air, the antenna does not conveniently support the portion between the antenna layer and the ground layer. Thus, buckling and instability can occur in the antenna layer subject to compression and tensile load.

In addition, since the antenna is typically manufactured to have a small size, a change in radiation characteristics must be considered. That is, radiation caused by transmission, diffraction, and reflection of electric waves occurring in or on the user's body must be considered. Radiation characteristics and reflection loss characteristics of the antenna may be determined by a medium included in the antenna. Therefore, various factors including characteristics of a medium of the human body and positions of the device need to be complexly considered.

The antenna that is to be implanted in the human body or attached to a surface of skin needs to employ biocompatible materials that do not cause any undesirable effects to the human body. Also, the characteristics and performance of the material need to be considered. In addition, frequency-dependent characteristic according to electromagnetic anisotropy of the human body need to be considered.

### SUMMARY

In a general aspect, there is provided an antenna including an antenna surface; a ground plane; and an air layer comprising a porous structure disposed between the antenna surface and the ground plane.

The air layer supports the antenna surface and the ground plane in a vertical, horizontal, or diagonal direction.

The air layer may further include a support body comprising a geometric shape that controls a capacity of air in the air layer.

The air layer may further include a support body comprising a geometric shape that controls a strength provided by the air layer for supporting the antenna surface and the ground plane.

The air layer may control electrical characteristics of the antenna.

A surface area or a thickness of the air layer may control radiation efficiency of the antenna.

In another general aspect, there is provided a wireless physiological signal sensing device comprising an antenna surface connected to an antenna; a ground plane; and a support layer disposed between the antenna surface and the ground plane and comprising any one or any combination of a signal measurement unit, a signal processing unit, a wireless communication unit, and an air layer comprising a porous structure.

The air layer supports the antenna surface and the ground plane in a vertical, horizontal, or diagonal direction.

The air layer may further include a support body comprising a geometric shape that controls a capacity of air in the air layer.

The air layer may further include a support body comprising a geometric shape that controls a strength provided by the air layer for supporting the antenna surface and the ground plane.

The air layer may control electrical characteristics of the antenna.

A surface area or a thickness of the air layer may control radiation efficiency of the antenna.

In another general aspect, there is provided a method of manufacturing an antenna, the method including depositing a ground plane; depositing an air layer comprising a porous structure on the ground plane; and depositing an antenna surface on the air layer.

In another general aspect, there is provided a method of manufacturing a porous structure, the method including injecting polymer beads into polydimethylsiloxane (PDMS); and removing the polymer beads.

The method may further include curing the PDMS comprising the injected polymer beads, wherein the removing of the polymer beads comprises removing the cured polymer beads using acetone to form an air layer.

An air capacity and a strength of the air layer may be based on a size of the polymer beads.

In another general aspect, there is provided a method of manufacturing a porous structure, the method including generating air bubbles in polydimethylsiloxane (PDMS); curing the PDMS including the air bubbles; and applying an external air pressure to the PDMS to form an air layer.

The generating air bubbles in PDMS may include generating air bubbles in PDMS using ultrasonic waves or air, and the curing the PDMS including the air bubbles may include curing the PDMS including the air bubbles by emitting ultraviolet (UV) rays on the PDMS.

An air capacity of the air layer may be controlled by the applying of the external air pressure.

In another general aspect, there is provided a method of manufacturing a porous structure, the method including injecting polydimethylsiloxane (PDMS) in a mold comprising a protrusion; curing the PDMS; and removing the mold to form an air layer comprising a porous structure.

### BRIEF DESCRIPTION OF THE DRAWINGS

FIG. 1 is a diagram illustrating an example of an antenna.
FIGS. 2A and 2B are diagrams illustrating an example of a porous structure.
FIG. 3 is a diagram illustrating an example of a wireless physiological signal sensing device.
FIGS. 4A and 4B are diagrams illustrating examples of a method of manufacturing a porous structure.
FIGS. 5A, 5B, and 5C are diagrams illustrating examples of another method of manufacturing a porous structure.
FIGS. 6A, 6B, and 6C are diagrams illustrating examples of yet another method of manufacturing a porous structure.
FIG. 7 is a flowchart illustrating an example of a method of manufacturing an antenna.

### DETAILED DESCRIPTION

The following detailed description is provided to assist the reader in gaining a comprehensive understanding of the methods, apparatuses, and/or systems described herein. However, various changes, modifications, and equivalents of the systems, apparatuses and/or methods described herein will be apparent to one of ordinary skill in the art. Also, descriptions of functions and constructions that are well known to one of ordinary skill in the art may be omitted for increased clarity and conciseness.

Throughout the drawings and the detailed description, the same reference numerals refer to the same elements. The drawings may not be to scale, and the relative size, proportions, and depiction of elements in the drawings may be exaggerated for clarity, illustration, and convenience.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

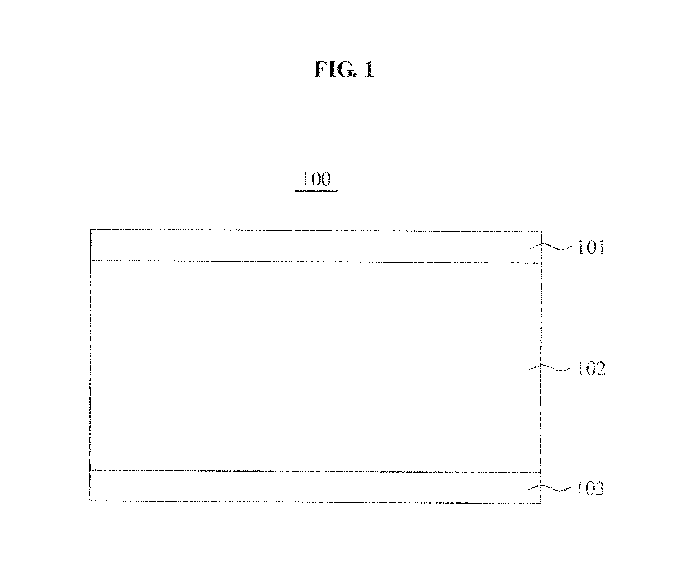

FIG. 1 illustrates an example of an antenna 100.

Referring to FIG. 1, the antenna 100 may include an antenna surface 101, an air layer 102, and a ground plane 103.

In this example, the ground plane 103 is attached to the skin of the user, the air layer 102 is disposed on the ground plane 103, and the antenna surface 101 is disposed on the air layer 102.

In an example, the air layer 102 has a porous structure. The porous structure of the air layer 102 may include a support body of a certain geometric shape. For example, the porous structure may include a circle having a uniform radius, an entangled fiber structure, or other structure. In this example, the support body may include air. The porous structure takes on a certain geometric shape in order to maximize air content.

The air layer 102 supports the antenna surface 101 and the ground plane 103 in vertical, horizontal, and diagonal directions. A supporting strength of the air layer 102 with respect to the antenna surface 101 and the ground plane 103 is adjusted according to the porous structure. That is, the air layer 102 may increase its air content according to the shape of the support body, thereby increasing the strength for supporting the antenna surface 101 and the ground plane 103.

When the support for the antenna surface 101 and the ground plane 103 is strengthened by the air layer 102, drooping or buckling of the antenna surface 101 is prevented. In addition, when the strength of the support body of the antenna 100 is reinforced, characteristics and performance of the antenna 100 are constantly maintained. As a result of preventing or reducing drooping or buckling of the antenna 100, a dielectric constant is reduced and radiation efficiency of the antenna 100 is increased.

The dielectric constant of the antenna 100 is reduced by increasing the air content of the air layer 102 having the porous structure. As a result, a surface area or thickness of the antenna 100 may be reduced and a contact efficiency of the antenna 100 with respect to the user's body may be increased. In addition, because the antenna 100 is divided into the antenna surface 101, the ground plane 103, and the air layer 102 and allows the surface area and thickness of each portion to be independently controlled, radiation efficiency may be increased.

In this example, the antenna 100 is a stable structure that is attached to the skin of the user and transmits physiological signals. The antenna 100 reduces influences of a high dielectric constant and conductivity of the human body, and may prevent reduction in impedance matching efficiency.

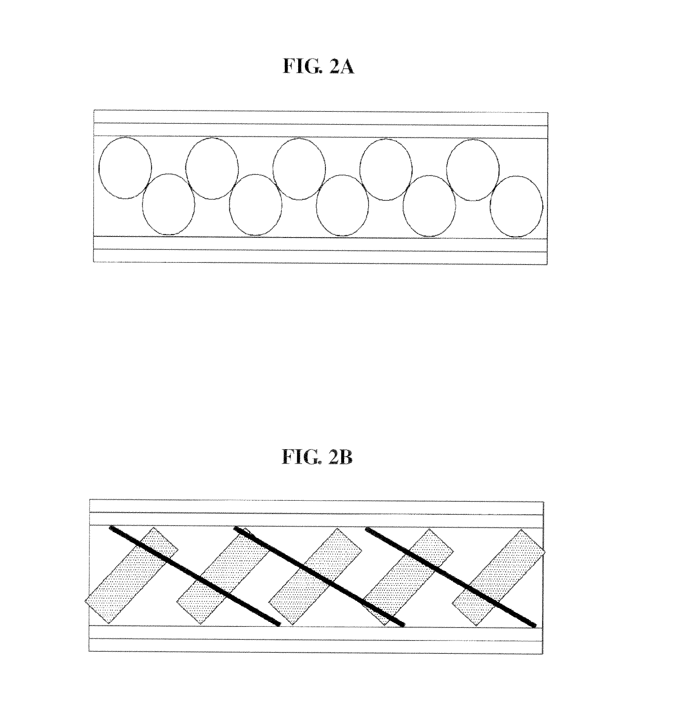

FIGS. 2A and 2B illustrate an example of a porous structure.

Referring to FIGS. 2A and 2B, an air layer may include a porous structure. The air layer adjusts air content by a support body having a geometric shape. Since the air layer reduces permittivity as a result of the increased air content, performance of the antenna is increased.

In an example, the porous structure of the air layer may include circles having a uniform radius as shown in FIG. 2A. The size of the each circle is adjusted based on the size of its radius. Therefore, the air content of the air layer is adjusted according to the size of the circles and the size of their radii. Additionally, the support strength of the air layer is adjusted according to the size and density of the circles. In this example, the strength of the air layer is increased in the vertical and horizontal directions based on the support body.

In another example shown in FIG. 2B, the porous structure of the air layer may include an entangled fiber structure. In this example, the air layer holds air through the fiber structure. Strength of the air layer is increased in a diagonal direction and the air layer maintains a constant distance between the antenna surface and the ground plane. As a result, performance of the antenna as expected may be maintained.

The porous structure of these examples may have various other forms and are not limited to the examples disclosed herein.

That is, according to various examples, the antenna may increase radiation efficiency by including an air layer having a porous structure. Also, the antenna reduces the influence of the high dielectric constant and conductivity of the human body. As a result, the reduction in the dielectric constant and conductivity may prevent reduction in impedance matching efficiency. The antenna reduces concentration of radiated power toward the human body by maintaining the distance between the antenna surface and the ground plane.

The surface area or thickness of the air layer may be controlled to adjust and increase the radiation efficiency of the antenna. That is, the surface area and thickness of the air layer may be controlled so that the radiation efficiency of the antenna may be maximized while user convenience and characteristics of the antenna are maintained.

For example, when the surface area or thickness of the antenna is reduced, the contact efficiency with respect to the human body may increase. That is, when the surface area or thickness is reduced, flexibility of the antenna may increase, thereby increasing the contact efficiency. Also, radiation efficiency may increase as a result of the reduced surface area or thickness.

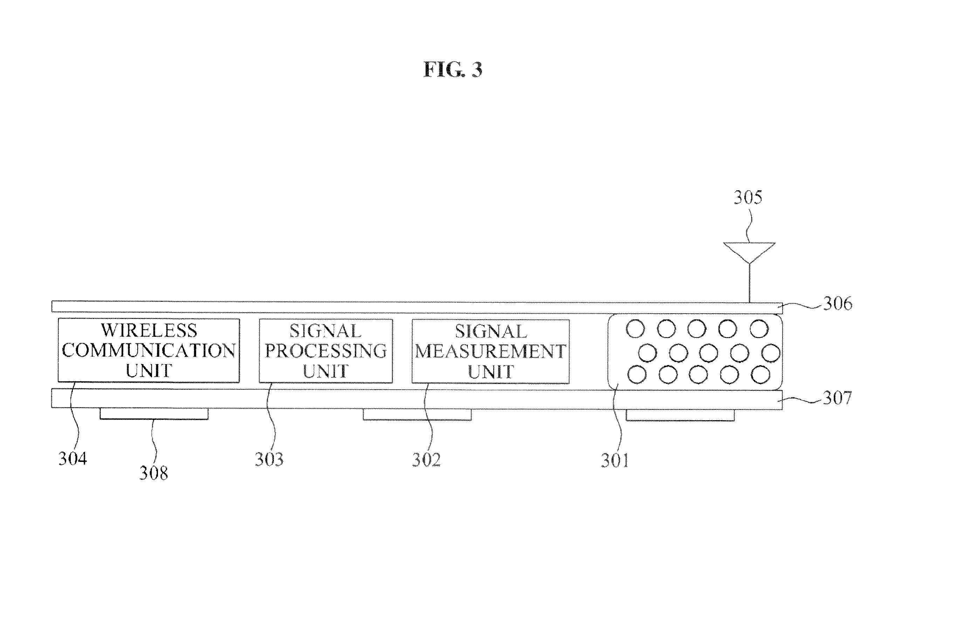

FIG. 3 illustrates an example of a wireless physiological signal sensing device.

Referring to FIG. 3, the wireless physiological signal sensing device may include an antenna 305, an antenna surface 306, a signal measurement unit 302, a signal processing unit 303, a wireless communication unit 304, an air layer 301 having a porous structure, a ground plane 307, and an electrode unit 308.

In this example, the antenna 305 is separately connected to the upper portion of the antenna surface 306. A support layer is provided between the antenna surface 306 and the ground plane 307. For example, the support layer may include the signal measurement unit 302, the signal processing unit 303, the wireless communication unit 304, and the air layer 301. The signal measurement unit 302, the signal processing unit 303, the wireless communication unit 304, and the air layer 301 are disposed adjacent to each other at predetermined intervals.

The wireless physiological signal sensing device may secure a maximum clearance of the human body by including the air layer 301 which has a porous structure. Since the antenna is disposed at an uppermost end of the wireless physiological signal sensing device, sensed physiological signals are efficiently transmitted and received. In addition, the wireless physiological signal sensing device may prevent electromagnetic waves from being absorbed by the human body.

In addition, the wireless physiological signal sensing device of this example may prevent drooping or buckling of the device as a result of the support provided by the air layer 301. Therefore, characteristics and performance of the wireless physiological signal sensing device are maintained. As the air content of the air layer 301 may increase, the strength of the air layer 301 for supporting the wireless physiological signal sensing device similarly may increase.

In this example, the surface area and thickness of the air layer 301 are adjusted to increase radiation efficiency. By adjusting the surface area and thickness of the air layer 301, the contact efficiency with respect to the body of the user similarly may increase. That is, as a result of the porous structure of the air layer 301, absorption of the electromagnetic waves by the human body may be minimized while radiation efficiency is maximized.

In this example, the wireless physiological signal sensing device is manufactured in such a manner that the signal measurement unit 302, the signal processing unit 303, the wireless communication unit 304, and the air layer 301 are prefabricated and separated by predetermined distances.

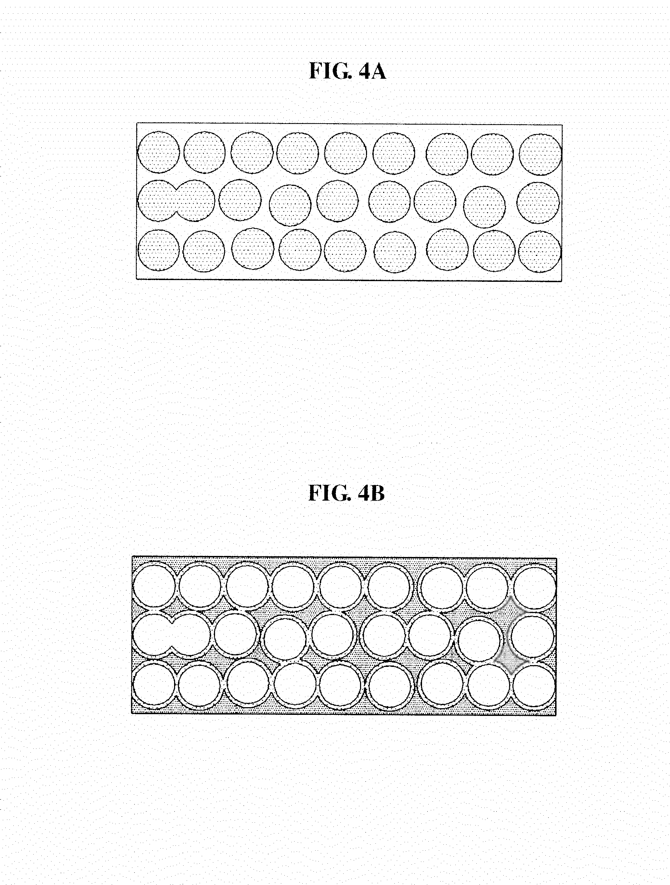

FIGS. 4A and 4B illustrate an example of a method of manufacturing the porous structure.

In an example, Polydimethylsiloxane (PDMS) is used in the porous structure manufacturing method for its various advantageous properties such as a loss tangent and flexibility when surface energy is low.

In this example of a manufacturing method of a porous structure, polymer beads are injected in the PDMS as shown in FIG. 4A. The PDMS may be in a mixture form. For example, the PDMS may be a mixture of Dow Corning Sylgard 184 prepolymer and a curing agent mixed at a ratio of 10:1. In this example, the polymer bead is injected by a volume ratio of approximately 1:1, and the PDMS including the injected polymer beads are cured. After the PDMS is cured, the polymer beads are removed using acetone. As a result, the PDMS may include spaces created by removing the polymer beads for holding air within the structure.

As shown in FIG. 4B, holes are formed in the PDMS corresponding to the polymer beads. The size of the holes may be adjusted according to a size of the polymer beads. As a result, the PDMS adjusts air capacity and strength according to the size of the holes. Similarly, adjusting the quantity of polymer beads allows adjustment of the number of holes which may increase the air capacity and strength of the porous PDMS. As the air content and the strength of the porous PDMS are increased, performance of the antenna may increase.

Additionally, the PDMS of this example reduces the loss tangent using the air layer having a porous structure. As a result of reducing the loss tangent, radiation efficiency of the antenna may increase. The loss tangent is controlled by the quantity of air within the air layer. That is, as the air content of the PDMS may increase, the loss tangent is reduced.

Accordingly, by increasing air capacity using the air layer having a geometric porous structure, the PDMS reduces the loss tangent and relative permittivity. Moreover, radiation efficiency may increase while the thickness may be reduced.

When the air capacity of the PDMS may increase, the antenna is stably supported in vertical, horizontal, and diagonal directions. Therefore, the properties and performance of the antenna are constantly maintained.

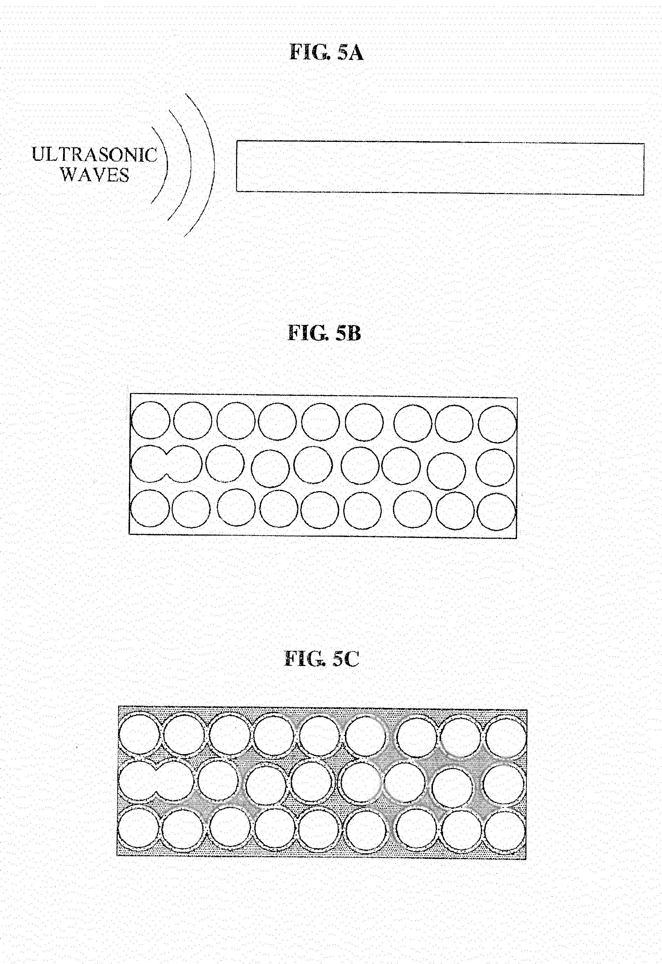

FIGS. 5A, 5B, and 5C illustrate examples of another method for manufacturing a porous structure.

In the example of FIG. 5A, ultrasonic waves or air are injected in the PDMS. For example, air may be injected using a pump. As shown in FIG. 5B, air bubbles are generated in the PDMS due to the injected ultrasonic waves or the air. In an example, the PDMS is in a mixture form. For example, the PDMS is a mixture of Gelest RMS-033 and Photoinitiator(2, 2-dimethoxy-2-phenylacetophenone) at a ratio of 10:0.1.

As shown in FIG. 5C, ultraviolet (UV) rays are emitted onto the PDMS and air bubbles therein. The PDMS is cured along with holes that include air. The air capacity of the PDMS is adjusted according to an external air pressure applied to the PDMS. The PDMS may increase the strength for supporting the antenna surface and the ground plane by increasing the its air capacity and content.

In this example, the dielectric constant of the PDMS is reduced in response to adjustments of the air capacity and content. For example, the radiation efficiency of the antenna may be made to increase. Also, the thickness of the antenna may be reduced as a result of reducing the dielectric constant. That is, as the air content of the air layer may increase, a weight or thickness of the antenna may be reduced.

When the air content of the PDMS may increase, internal energy efficiency also may increase because surface energy is low. That is, the PDMS may increase radiation efficiency by increasing air capacity and content within the antenna.

In this example, an antenna including PDMS has a stable structure that allows receiving physiological signals while being attached to the skin of the user. For example, the antenna reduces the high dielectric constant and conductivity of the human body. Furthermore, the reduction in the impedance matching efficiency is also prevented.

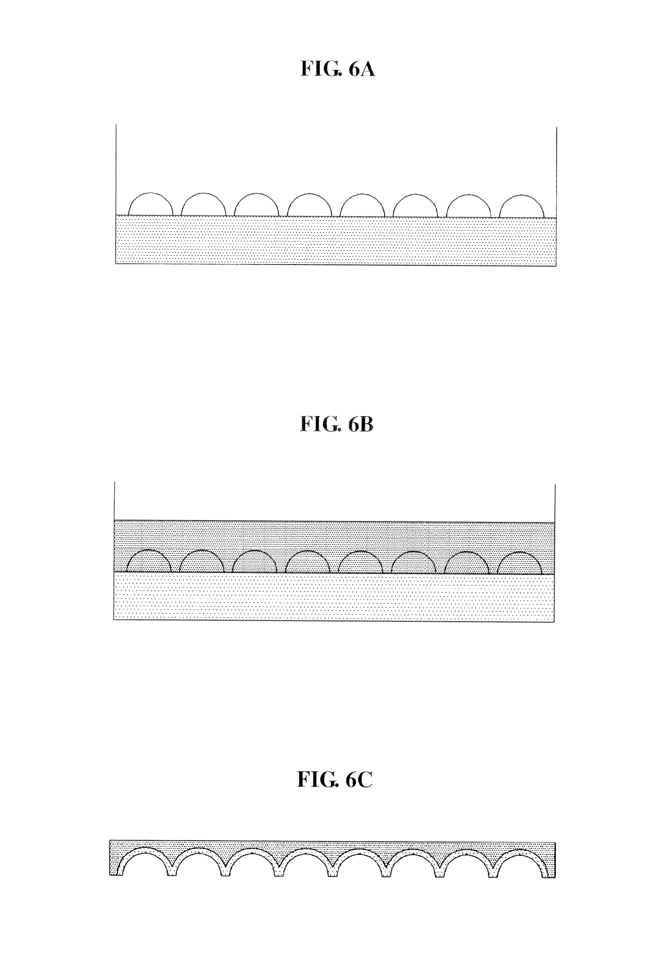

FIGS. 6A, 6B, and 6C illustrate examples of yet another method for manufacturing a porous structure.

As shown in FIG. 6A, a mold for receiving PDMS may include one or more protrusions having variable sizes. In the step shown in FIG. 6B, PDMS is mixed and injected into the mold. For example, the PDMS is a mixture of Dow Corning Sylgard 184 prepolymer and a curing agent mixed at a ratio of 10:1. The PDMS is then cured and the mold is removed as shown in FIG. 6C.

When the mold is removed, the PDMS takes the shape of the one or more protrusions of the mold. Accordingly, the PDMS may include spaces corresponding to the protrusions that are capable of carrying air. Thus, a maximum amount of air may be included in the PDMS in order to increase the radiation efficiency of the antenna. In addition, the PDMS supports the antenna surface and the ground plane.

In this example, the PDMS may prevent drooping or buckling of the antenna by increasing the strength for supporting the antenna surface and the ground plane. Because the strength is increased, the property and the performance of the antenna are maintained. As a result of the air layer with porous structure included in the PDMS, structural stability of the antenna is enhanced. Furthermore, as the air capacity and content may increase, the strength for supporting the antenna similarly may increase.

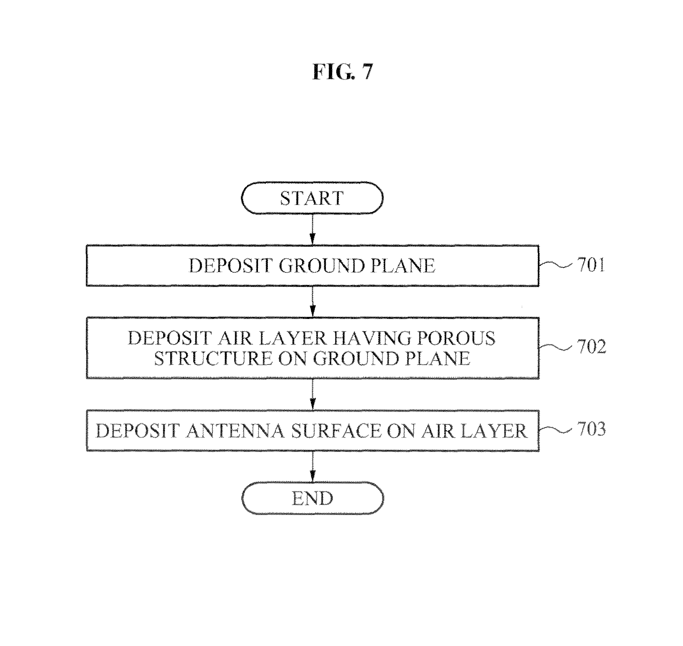

FIG. 7 illustrates an example of method for manufacturing an antenna.

In operation 701, a ground plane of the antenna is deposited.

In operation 702, an air layer having a porous structure is deposited on the ground plane. The presence of the air layer within the antenna allows for the thickness of the antenna to be minimized. The air layer has a porous structure that may include a support body with a certain geometric shape. The support body may include air. For example, the support body may have one or more geometric shapes such as a circle having a uniform radius, an entangled fiber structure, or other shapes. Additionally, it should be appreciated that the descriptions provided above regarding the examples of porous air layers are applicable for this example.

In operation 703, the antenna surface is deposited on the air layer. The antenna is attached to the human body for measuring physiological signals. In this example, the antenna may increase in strength and stability resulting from the porous structure of the air layer. When the strength of the antenna is maintained, the dielectric constant is reduced while the radiation efficiency is increased. Further, the antenna generates a radiation pattern that is concentrated in a direction opposite to the user's body, thereby reducing communication error caused by the high dielectric constant and conductivity of the body. , Reduction in impedance matching efficiency is prevented as a result of reducing the dielectric constant and conductivity. Further, radiation efficiency of the antenna is increased and strength for supporting the antenna is maintained. In this example, the antenna may be an ultra-thin and flexible antenna having a low dielectric constant and regular performance. The antenna efficiently monitors physiological signals of the user by being attached to the body of the user. In an example, the antenna may be a wearable antenna having an ultra-thin and flexible structure and including a dielectric body through the porous structure.

The antenna 305, antenna surface 306, signal measurement unit 302, signal processing unit 303, wireless communication unit 304, air layer 301, ground plane 307, and electrode unit 308 described above may be implemented using one or more hardware components, or a combination of one or more hardware components and one or more software components. A hardware component may be, for example, a physical device that physically performs one or more operations, but is not limited thereto. Examples of hardware components include controllers, microphones, amplifiers, low-pass filters, highpass filters, band-pass filters, analog-to-digital converters, digital-to-analog converters, and processing devices.

A processing device may be implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instructions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

Software or instructions for controlling a processing device, such as those described in FIG. 7, to implement a software component may include a computer program, a piece of code, an instruction, or some combination thereof, for independently or collectively instructing or configuring the processing device to perform one or more desired operations. The software or instructions may include machine code that may be directly executed by the processing device, such as machine code produced by a compiler, and/or higher-level code that may be executed by the processing device using an interpreter. The software or instructions and any associated data, data files, and data structures may be embodied permanently or temporarily in any type of machine, component, physical or virtual equipment, computer storage medium or device, or a propagated signal wave capable of providing instructions or data to or being interpreted by the processing device. The software or instructions and any associated data, data files, and data structures also may be distributed over network-coupled computer systems so that the software or instructions and any associated data, data files, and data structures are stored and executed in a distributed fashion.

For example, the software or instructions and any associated data, data files, and data structures may be recorded, stored, or fixed in one or more non-transitory computer-readable storage media. A non-transitory computer-readable storage medium may be any data storage device that is capable of storing the software or instructions and any associated data, data files, and data structures so that they can be read by a computer system or processing device. Examples of a non-transitory computer-readable storage medium include read-only memory (ROM), random-access memory (RAM), flash memory, CD-ROMs, CD-Rs, CD+Rs, CD-RWs, CD+RWs, DVD-ROMs, DVD-Rs, DVD+Rs, DVD-RWs, DVD+RWs, DVD-RAMs, BD-ROMs, BD-Rs, BD-R LTHs, BD-REs, magnetic tapes, floppy disks, magneto-optical data storage devices, optical data storage devices, hard disks, solid-state disks, or any other non-transitory computer-readable storage medium known to one of ordinary skill in the art.

Functional programs, codes, and code segments for implementing the examples disclosed herein can be easily constructed by a programmer skilled in the art to which the examples pertain based on the drawings and their corresponding descriptions as provided herein.

While this disclosure may include specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is defined not by the detailed description, but by the claims and their equivalents, and all variations within the scope of the claims and their equivalents are to be construed as being included in the disclosure.

## Claims

1. An antenna attached to a user or implanted in a user's body to transmit physiological signals comprising:
an antenna surface
a ground plane; and
an air layer comprising a porous structure disposed between the antenna surface and the ground plane.

2. The antenna of claim 1, wherein the air layer supports the antenna surface and the ground plane in a vertical, horizontal, or diagonal direction.

3. The antenna of claim 1 or 2, wherein the air layer further comprises a support body comprising a geometric shape that controls a capacity of air in the air layer.

4. The antenna of one of the previous claims, wherein the air layer further comprises a support body comprising a geometric shape that controls a strength provided by the air layer for supporting the antenna surface and the ground plane.

5. The antenna of one of the previous claims, wherein the air layer controls electrical characteristics of the antenna.

6. The antenna of one of the previous claims, wherein a surface area or a thickness of the air layer controls radiation efficiency of the antenna.

7. A wireless physiological signal sensing device comprising an antenna according to one of the claims 1 to 4
said antenna surface connected to an antenna;
a ground plane; and
a support layer disposed between the antenna surface and the ground plane and comprising any one or any combination of a signal measurement unit, a signal processing unit, a wireless communication unit, and an air layer comprising a porous structure.

8. A method of manufacturing a porous structure of an antenna according to claims 1 to 6, the method comprising:
injecting polymer beads into polydimethylsiloxane (PDMS); and
removing the polymer beads.

9. The method of claim 8, further comprising
curing the PDMS comprising the injected polymer beads, wherein
the removing of the polymer beads comprises removing the cured polymer beads using acetone to form an air layer.

10. The method of claim 8 or 9, wherein an air capacity and a strength of the air layer is based on a size of the polymer beads.

11. A method of manufacturing a porous structure of an antenna according to claims 1 to 6, the method comprising:
generating air bubbles in polydimethylsiloxane (PDMS);
curing the PDMS including the air bubbles; and
applying an external air pressure to the PDMS to form an air layer.

12. The method of claim 11, wherein the generating air bubbles in PDMS comprises generating air bubbles in PDMS using ultrasonic waves or air, and the curing the PDMS including the air bubbles comprises curing the PDMS including the air bubbles by emitting ultraviolet (UV) rays on the PDMS.

13. The method of claim 11 or 12, wherein an air capacity of the air layer is controlled by the applying of the external air pressure.

14. A method of manufacturing a porous structure of an antenna according to claims 1 to 6, the method comprising:
injecting polydimethylsiloxane (PDMS) in a mold comprising a protrusion;
curing the PDMS; and
removing the mold to form an air layer comprising a porous structure.
